# EUROPEAN PATENT APPLICATION

(11) **EP 0 724 882 A1**
(43) Date of publication of application: **07.08.1996**
(21) Application number: 95926009.2
(22) Date of filing: 21.07.1995
(51) Int. Cl.: A61K 31/70, C07H 21/02, C07H 21/04

(54) **NOVEL ANTI-HIV DRUG**

(30) Priority: 22.07.1994 JP 200029/94; 22.07.1994 JP 200030/94; 22.07.1994 JP 200031/94; 22.07.1994 JP 200032/94; 26.08.1994 JP 238313/94; 26.08.1994 JP 238314/94; 26.08.1994 JP 238315/94; 26.08.1994 JP 238316/94; 26.08.1994 JP 238317/94
(71) Applicant: KAJI, Akira, Tokyo 203 (JP)
(72) Inventor: KAJI, Hideko, Tokyo 203 (JP)
(74) Representative: Boeters, Hans Dietrich, Dr.
(86) International application number: JP9501456
(87) International publication number: WO9603133

(57) **Abstract**

An anti-HIV agent which comprises as an active ingredient one or more species selected from phosphodiester bonding type or phosphorothioester bonding type oligoribonucleotides with 2-8 bases represented by the 4 bases of the below formulae (III), (VII), (XI), (XVII) and (XXI)
GpGpGpGp (III)
pGpGpGpG (VII)
GsGsGsGp (XI)
pGsGsGsG (XVII)
GsGsGsG (XXI)
wherein G represents guanosine structure, p is a phosphoric acid residue which forms an ester bonding with neighboring G, and s is the thiophosphoric acid residue which forms the phosphorothioester bonding with neighboring G.

## Description

### Field of the Industrial Application

The present invention relates to an anti-HIV (Human Immunodeficiency Virus) agent containing as an active ingredient a phosphodiester bonding type or phosphorothioester bonding type oligoribonucleotide which shows an excellent anti-viral action for diseases caused by HIV.

### Background Art

As a previous finding on homooligomers of DNA, Agrawal et al. reported that the phosphorothioates with chain lengths of 15 bases and 20 bases were active for inhibition of HIV replication, and the effect was due to homooligomer with chain length of the 15-bases and was independent of a kind of the base. As for the effectiveness, it was also reported that due to the lengthy homooligomers they might favorably act as a template of reverse transcriptase and be competitive to HIV nucleic acid (Proc. Natl. Acad. Sci. USA, 85, 7079, 1988) .

Additionally, Matsukura et al. reported that the deoxycytidine phosphorothioate binding compound of 28-bases had an activity for inhibition of HIV replication (Proc. Natl. Acad. Sci. USA, 84, 7706, 1987) .

As a previous finding on oligoribonucleotide, furthermore, Shibahara et al. showed that the 2'-o-methylinosine phosphorothioate of 20-bases had the activity for inhibition of HIV replication (Nucleic Acids Res., 17, 239, 1989), and Crawford et al. reported that polyadenine - polyuridine also had the activity for inhibition of HIV replication (AIDS Res. Human Retroviruses. 8, 285, 1992). However, since all these are long chain nucleotides with the length of 14 - 28 bases or more, there are problems not only in the preparation, but also in the absorption and stability when they are administered into a body.

In order to solve these problems, the inventors made an extensive research on obtaining a low molecular oligonucleotide with the activity for inhibition of HIV replication, and recently found that a short chain oligodeoxyguanylic acid of 4-8 bases had the activity for inhibition of HIV replication.

As a result of a further study, the inventors newly found that a short chain oligoriboguanylic acid of 2-8 bases had the activity for inhibition of HIV replication. The invention is based on this finding.

Accordingly, the invention provides an anti-HIV agent characterized in that it comprises as an active ingredient one or more species selected from phosphodiester bonding type oligoribonucleotides or phosphorothioester bonding type oligoribonucleotides represented by the below formulae (I)-(XXIII) described below in the item's a)-e).
a) An anti-HIV agent which comprises as an active ingredient one or more species selected from phosphodiester bonding type oligoribonucleotides represented by either of the below formulae (I)-(IV)
   GpGp (I)
   GpGpGp (II)
   GpGpGpGp (III)
   GpGpGpGpGp (IV)
   wherein G represents guanosine structure, p is a phosphoric acid residue which forms an ester bonding with neighboring G, each right terminal p forms the monoester bonding with 3'-hydroxyl of 3'-terminal G, each in-between p of two Gs forms the ester bondings with 3'-hydroxyl of the left side G and with 5'-hydroxyl of the right side G in the formulae respectively.
b) An anti-HIV agent which comprises as an active ingredient one or more species selected from phosphodiester bonding type oligoribonucleotides represented by either of the below formulae (V)-(VIII)
   pGpG (V)
   pGpGpG (VI)
   pGpGpGpG (VII)
   pGpGpGpGpG (VIII)
   wherein each G and p have the meaning indicated, and each left terminal p forms the monoester bonding with 5'-hydroxyl of 5'-terminal G.
c) An anti-HIV agent which comprises as an active ingredient one or more species selected from phosphorothioester bonding type oligoribonucleotides represented by either of the below formulae (IX)-(XV)
   GsGp (IX)
   GsGsGp (X)
   GsGsGsGp (XI)
   GsGsGsGsGp (XII)
   GsGsGsGsGsGp (XIII)
   GsGsGsGsGsGsGp (XIV)
   GsGsGsGsGsGsGsGp (XV)
   wherein each G and p have the meaning indicated, and s is the thiophosphoric acid residue which forms the phosphorothioester bonding with neighboring G and each s forms the ester bondings with 3'-hydroxyl of the left side G and with 5'-hydroxyl of the right side G in the formulae respectively.
d) An anti-HIV agent which comprises as an active ingredient one or more species selected from phosphorothioester bonding type oligoribonucleotides represented by either of the below formulae (XVI)-(IXX)
   pGsGsG (XVI)
   pGsGsGsG (XVII)
   pGsGsGsGsG (XVIII)
   pGsGsGsGsGsG (IXX)
   wherein G, p and s have the meaning indicated respectively.
e) An anti-HIV agent which comprises as an active ingredient one or more species selected from phosphorothioester bonding type oligoribonucleotides represented by either of the below formulae (XX)-(XXIII)
   GsGsG (XX)
   GsGsGsG (XXI)
   GsGsGsGsG (XXII)
   GsGsGsGsGsG (XXIII)
   wherein G and s have the meaning indicated respectively.

In the following, the invention is described in detail.

The oligoribonucleotide of the above formulae (I)-(XXIII) which is an active ingredient of the anti-HIV agent of the invention, can generally be synthesized by usual methods (see Scaringe et al., Nucleic Acids Res. 18, 5433; Damha and Ogilvie, Methods in Mol. Biol. 20 81; Eckstein, Ann. Rev. Biochem. 54, 367). A favorable and representative synthetic method as a synthetic method for the compounds of the invention is illustrated as [Synthetic method A] - [Synthetic method E] in the followings.

### [Synthetic method A]

The material (P35, Glen Research User Guide, published by Glen Research Institute, 44901 Falcon Place, Sterling, VA 20166) in which 2-[2-(4,4'-dimethoxytrityloxy)-ethylsulfonyl] succinoyl long chain alkyl amino group is bound to controlled-pore glass (CPG) as a support, is prepared. After the protective 4,4'-dimethoxytrityl group is deprotected by trichloroacetic acid, etc. , the amidite reagent, (5'-o-dimethoxytrityl)-2'-o-(t-butyldimethylsilyl)-ribonucleoside-3'-N, N-diisopropyl(cyanoethyl)-phosphoramidite, is subjected to the condensation by tetrazole in which the amino group in guanylic acid base as a monomer is protected by isobutyl group. This is followed by the oxidation of the phosphorous part with iodine to lead a phosphoric acid triester derivative. The above reaction is repeated until an aimed chain length is obtained. An ammonia treatment then converts each internucleotide from the triester to the diester and carries out the detachment from the support and the simultaneous deblocking of the base part. This is followed by a tetra-N-butylammonium fluoride treatment to deblock 2'-hydroxyl protecting group giving an aimed oligoribonucleotide phosphorylated at the 3'-terminal position in this method.

### [Synthetic method B]

In a case of the solid phase synthesis by the phosphoramidate method, a material in which a functionally protected guanosine is bound to a support such as controlled-pore glass (CPG), is prepared. After the protective 4,4'-dimethoxytrityl group of 5'-hydroxyl in this nucleoside is deprotected by trichloroacetic acid, etc. , the amidite reagent, (5'-o-dimethoxytrityl)-2'-o-(t-butyldimethylsilyl) ribonucleoside-3'-N, N-diisopropyl (cyanoethyl)phosphoramidite, is subjected to the condensation by tetrazole in which the amino group of guanylic acid base as a monomer is protected by isobutyl group. To avoid a by-product production, the unreacted 5'-hydroxyl group is acetylated by acetic anhydride and N-methylimidazole, etc. This is followed by the oxidation of the phosphorous part with iodine to lead a phosphoric acid triester derivative. The above reaction is repeated until an aimed chain length is obtained. Further, the phosphorylation reagent, (2-cyanoethoxy)-2-(2'-o-4,4'-dimethoxytrityloxyethyl-sulfonylethoxy-N,N-diisopropyl aminophosphine, is bound to the 5'-terminal hydroxyl group (see Horn & Urdea, Tetrahedron Letters 27, 4705; Uhlmann & Engels, Tetrahedron Letters 27, 1023). This is followed by the oxidation of the terminal phosphorous part with iodine to lead a phosphoric acid triester. After this, an ammonia treatment converts each internucleotide from the triester to the diester and carries out the detachment from the support and the simultaneous deblocking of the base part. This is followed by a tetra-N-butylammonium fluoride treatment to deblock 2'-hydroxyl protecting group giving an aimed oligoribonucleotide phosphorylated at the 5'-terminal position in this method.

### [Synthetic method C]

The material (see the publication of Glen Research Institute described above) in which 2-[2-(4,4'-dimethoxytrityloxy)ethylsulfonyl]succinoyl long chain alkyl amino group is bound to controlled-pore glass (CPG) as a support, is prepared. After the protective 4,4'-dimethoxytrityl group is deprotected by trichloroacetic acid, etc. , the amidite reagent, (5'-o-dimethoxytrityl)-2'-o-(t-butyldimethylsilyl) ribonucleoside-3'-N,N-diisopropyl (cyanoethyl) phosphoramidite, is subjected to the condensation by tetrazole in which the amino group in guanylic acid base as a monomer is protected by isobutyl group. This is followed by the oxidation of the phosphorous part with iodine to lead to a phosphoric acid triester derivative. The 5' protecting group (DMTr) of this guanylic acid is then removed by trichloroacetic acid, and as above is condensed the amidite reagent in which functional groups of the second guanylic acid are protected. This is followed by the sulfiding of the second phosphorous part with tetraethylthiuram disulfide to lead to a phosphorothiotriester. After the condensation of guanylic acid and the sulfiding of the phosphorous part are repeated until an aimed chain length is obtained, an ammonia treatment converts each internucleotide from the triester to the diester and carries out the detachment from the support and the simultaneous deblocking of the base part. This is followed by a tetra-N-butylammonium fluoride treatment to deblock 2'-hydoxyl protecting group giving an aimed oligoribonucleotide phosphorylated at the 3'-terminal position in this method.

### [Synthetic method D]

In this item, the phosphoramidite method is explained in case of obtaining oligoribonucleotide with a phosphorothioester bonding. A material in which a functionally protected guanosine is bound to a support such as controlled-pore glass (CPG), is prepared. After the protective 4,4'-dimethoxytrityl group of this nucleoside's 5'-hydroxyl is deprotected by trichloroacetic acid, etc. , the amidite reagent, (5'-o-dimethoxytrityl-2')-o-(t-butyldimethylsilyl) ribonucleoside-3'-N, N-diisopropyl (cyanoethyl) phosphoramidite, is subjected to the condensation by tetrazole in which the amino group of guanylic acid base is protected by isobutyl group. To avoid a by-product production, the unreacted 5'-hydroxyl group is acetylated by acetic anhydride and N-methylimidazole, etc. This is followed by the sulfiding of the phosphorous part with tetraethylthiuram disulfide to lead to a phosphoro-thiotriester. The above reaction is repeated until an aimed chain length is obtained, and is followed by deblocking the 5'-hydroxyl protecting group and by the condensation with the phosphorylation reagent, (2-cyanoethoxy)-2-(2'-o-4,4'-di-methoxytrityloxyethylsulfonyl)-ethoxy-N,N-diisopropyl amino-phosphine (see Tetrahedron Letters cited above). The oxidation of the terminal phosphorous part with iodine then gives a phosphoric acid triester. After this, an ammonia treatment converts the triester to the diester and carries out the detachment from the support and the simultaneous deblocking of the base part. This is followed by a tetra-N-butylammonium fluoride treatment to deblock 2'-hydroxyl protecting group giving an aimed phosphorothioate oligoribonucleotide phosphorylated at the 5'-terminal position in this method.

### [Synthetic method E]

In this item is explained the phosphoramidite method applied preferably to the synthesis of the compounds of the formulae (XX)-(XXIII). A material in which a functionally protected guanosine is bound to a support such as controlled-pore glass (CPG), is prepared. After the protective 4,4'-dimethoxytrityl group of this nucleoside's 5'-hydroxyl is deprotected by trichloroacetic acid, etc. , the amidite reagent, (5'-o-dimethoxytrityl)-2'-o-(t-butyldimethylsilyl) ribonucleoside-3'-N, N-diisopropyl(cyanoethyl)phosphoramidite, is subjected to the condensation by tetrazole in which the amino group of guanylic acid base is protected by isobutyl group. To avoid a by-product production, the unreacted 5'-hydroxyl group is acetylated by acetic anhydride and N-methylimidazole, etc. This is followed by the sulfiding of the phosphorous part with tetraethylthiuram disulfide to lead to a phosphorothiotriester. After the above reaction is repeated until an aimed chain length is obtained, an ammonia treatment converts each internucleotide from the triester to the diester and carries out the detachment from the support and the simultaneous deblocking of the base part. This is followed by a tetra-N-butylammonium fluoride treatment to deblock 2'-hydroxyl protecting group giving an aimed oligoribonucleotide in this method.

### Experimental examples

Experimental examples are given below to explain the invention in more details, but the invention is not limited thereby in any way.

### 〈Anti-AIDS virus (HIV) activity tests using MT-4 cell (T cell system)〉

To a 96-well microplate which contained each phosphodiester bonding type or phosphorothioester bonding type oligoribonucleotide previously diluted stepwise were placed MT-4 cells (6 x 10⁴ cells per well). The cells were then infected with AIDS virus [HIV-1(IIIB) : ca. 50 TCID₅₀ (50% tissue culture infectious doses)/well] and were incubated in a 5% CO₂ atmosphere at 37°C for 5 days. For evaluation of the inhibition of the cytopathic effect by the infection, viable cells were measured by MTT method after 5 days' incubation. The results are shown in Table 1.

In the formula of each oligoribonucleotide in the table, the meaning of p and s is identical to the definition described above, and G, A, C and U are guanosine, adenosine, cytidine and uridine respectively.

**Table 1**

| | ED₅₀ (50% cytopathic inhibitory concentration) |
|---|---|
| GpG | >100µg/ml |
| GpGpG | >100µg/ml |
| GpGpGpG | >100µg/ml (40% cytopathic inhibition) |
| ApApApAp | >100µg/ml |
| UpUpUpUp | >100µg/ml |
| CpCpCpCp | >100µg/ml |
| pGpG | >100µg/ml (25% cytopathic inhibition) |
| GpGp | 22.7µg/ml |
| GsGp | 28.5µg/ml |
| GsGsGp | 1.3µg/ml |
| pGsGsG | 5.3µg/ml |
| GsGsG | 16.6µg/ml |
| GpGpGp | 20.2µg/ml |
| pGpGpG | 45.1µg/ml |
| pGpGpGpG | 15.3µg/ml |
| GsGsGsGp | 0.7µg/ml |
| GpGpGpGp | 2.2µg/ml |
| pGsGsGsG | 1.1µg/ml |
| GsGsGsG | 4.0µg/ml |
| GpGpGpGpGp | 1.6µg/ml |
| pGsGsGsGsGsG | 0.9µg/ml |
| GsGsGsGsGsG | 2.0µg/ml |
| GsGsGsGsGsGp | 2.3µg/ml |
| GsGsGsGsGsGsGsGp | 1.9µg/ml |

As shown in Table 1, the phosphodiester bonding type or phosphorothioester bonding type oligoribonucleotides which are the active ingredients of the anti-HIV agents of the invention showed the effect even from 2 bases, and in 3-8 bases the excellent anti-HIV activity was shown. Further, no cytotoxicity was shown at the concentration of 100µg/ml in any oligoribonucleotide. On one hand, a phosphodiester bonding type oligoguanylic acid whose terminal G is not phosphorylated had no effect in 2 or 3 bases and showed a little effect in 4 bases. Each phosphodiester bonding type oligoadenylic acid, cytidylic acid and uridylic acid phosphorylated at 3'-terminal also showed no effect in 4 bases. On the contrary, in the phosphodiester bonding type and phosphorothioester bonding type oligoguanylic acids phosphorylated at 3'-terminal or 5'-terminal, their effectiveness is recognized to be remarkable.

As to the safety of each oligoguanylic acid which is the active ingredient of the anti-HIV agent of the invention, the safety was ascertained by an intravenous injection of 20mg/kg in mice using the compounds bound with 4 bases. In another experiment, it was also shown that no death was observed by an oral administration of each of these compounds at the dose of 100mg/kg.

The novel anti-HIV agents of the invention are useful in particular as a medicament for treating diseases infected by a retrovirus such as AIDS, and can be processed with pharmaceutical vehicles to oral preparations such as tablets, capsules, granules, fine granules and powders, or to non-oral preparations such as injections, i.v. drip infusions and suppositories.

As for an administration form there is no specification and a proper form can be selected and used as required. Illustrative of the oral preparations are, for example, tablets, capsules, granules, fine granules and powders, and those of the non-oral preparations are injections and suppositories.

The required dose as oral preparations depends on the age and body weight of each patient and the severity of the disease, though, the daily dose for adults is usually between 0.1-6 g which are favorably administered in several times.

The oral preparations such as the above tablets, capsules and granules can be prepared by the conventional method using, for example, starch, lactose, sugar, mannite, carboxymethylcellulose or inorganic salts.

In this kind of preparations, except to the above excipients other additives such as binders, disintegraters, surfactants, lubricants, flowing promoters, sweeteners, colorings and flavors can be selected and used appropriately. Each example is as shown below.

### [Binders]

Starch, dextrin, Arabic gum powder, gelatin, hydroxypropylstarch, methylcellulose, sodium carboxymethylcellulose, hydroxypropylcellulose, crystalline cellulose, ethylcellulose, polyvinylcellulose, macrogol.

### [Disintegraters]

Starch, hydroxypropylstarch, sodium carboxymethylcellulose, calcium carboxymethylcellulose, carboxymethylcellulose, low substituted hydroxypropylcellulose.

### [Surfactants]

Sodium laurylsulfate, soya lecithin, sucrose fatty acid ester, polysorvate 80.

### [Lubricants]

Talc, wax, hydrogenated vegetable oil, sucrose fatty acid ester, magnesium stearate, polyethylene glycol.

### [Flowing promoters]

Light anhydrous silicic acid, dry aluminum hydroxide gel, synthetic aluminum silicate, magnesium silicate.

The oligoribonucleotides of the formulae (I) - (XXIII) of the invention can be administered as suspensions, liquid emulsions, syrups or elixirs. For each dosage form of these, flavorings or colorings can be included.

The required dose as non-oral preparations depends on the age and body weight of each patient and the severity of the disease, though, the daily dose of the above oligoribonucleotides for adults is usually between 1-100mg which are administered by intravenous injection, i.v. drip infusion, subcutaneous injection or intramuscular injection.

These non-oral preparations can be prepared by the conventional method. In this case, as diluents can generally be used distilled water for injection, physiological saline solution, aqueous glucose solution, vegetable oil for injection, sesame oil, peanut oil, soybean oil, corn oil, propylene glycol, polyethylene glycol or the like. The preparations can contain antiseptics, preservatives or stabilizers as required. From the view point of stability, the non-oral preparations are filled into vials or, etc., and frozen and dried by the conventional lyophilizing technology. Liquid preparations can be prepared again just before use by using this lyophilizates. Further, there may be appropriately added isotonic agents, stabilizers, preservatives, soothing agents or, etc. , as required.

As the other non-oral preparations, embrocations such as externally applicable liquids and ointments, and suppositories for rectal administration can be illustrated and all these can be prepared by a conventional method.

### Examples

Examples are given below to explain the invention in more details, but the invention is not limited thereby in any way.

### Example 1

| Formulation: | |
|---|---|
| ① Crystalline cellulose | 84.5 wt. parts |
| ② Magnesium stearate | 0.5 wt. parts |
| ③ Calcium carboxymethylcellulose | 5 wt. parts |
| ④ GpGpGpGp | 10 wt. parts |
| | Total 100 wt. parts |

According to the above formulation, ①, ④ and a part of ② were mixed until they become uniform. The mixture was compressed and then pulverized to powder which was mixed with the remaining ② and ③. The mixture was compressed to form tablets (200mg/tablet) by a tablet machine.

In this one tablet, 20mg of GpGpGpGp is included and 4 - 8 tablets per day for adults are administered in several times.

### Example 2

| Formulation: | |
|---|---|
| ① Crystalline cellulose | 34.5 wt. parts |
| ② 10% ethanolic hydroxypropylcellulose solution | 50 wt. parts |
| ③ Calcium carboxymethylcellulose | 5 wt. parts |
| ④ Magnesium stearate | 0.5 wt. parts |
| ⑤ pGpGpGpG | 10 wt. parts |
| | Total 100 wt. parts |

According to the above formulation ①, ② and ⑤ were mixed until they become a uniform blend. The blend was kneaded by a conventional method and granulated by a pressing granulation machine, and after drying and pulverization the powder was mixed with ③ and ④. The mixture was compressed to form tablets (200mg/tablet) by a tablet machine.

In this one tablet, 20mg of pGpGpGpG is included and 4-8 tablets per day for adults are administered in several times.

### Example 3

| Formulation: | |
|---|---|
| ① Distilled water for injection | appropriate |
| ② Glucose | 200 mg |
| ③ GpGpGpGp | 10 mg |
| | Total 15 ml |

In the distilled water for injection ① were dissolved ② and ③ . The solution was filled into 5ml ample which was sterilized at 121°C for 15 min. to afford injections.

Further, the other compounds except the compounds described in the above each example can also be processed to the preparations of every kind of dosage form according to the formulations shown in the above each example. Effect of the Invention

The novel anti-HIV agents of the invention are effective for the treatment of the HIV (AIDS virus) induced diseases such as AIDS and ARC.

## Claims

1. An anti-HIV agent which comprises as an active ingredient one or more species selected from phosphodiester bonding type oligoribonucleotides represented by either of the below formulae (I) -(IV)
GpGp (I)
GpGpGp (II)
GpGpGpGp (III)
GpGpGpGpGp (IV)
wherein G represents guanosine structure, p is a phosphoric acid residue which forms an ester bonding with neighboring G, each right terminal p forms the monoester bonding with 3'-hydroxyl of 3'-terminal G, each in-between p of two Gs forms the ester bondings with 3'-hydroxyl of the left side G and with 5'-hydroxyl of the right side G in the formulae respectively.

2. An anti-HIV agent which comprises as an active ingredient one or more species selected from phosphodiester bonding type oligoribonucleotides represented by either of the below formulae (V)-(VIII)
pGpG (V)
pGpGpG (VI)
pGpGpGpG (VII)
pGpGpGpGpG (VIII)
wherein each G and p have the meaning indicated, and each left terminal p forms the monoester bonding with 5'-hydroxyl of 5'-terminal G.

3. An anti-HIV agent which comprises as an active ingredient one or more species selected from phosphorothioester bonding type oligoribonucleotides represented by either of the below formulae (IX)-(XV)
GsGp (IX)
GsGsGp (X)
GsGsGsGp (XI)
GsGsGsGsGp (XII)
GsGsGsGsGsGp (XIII)
GsGsGsGsGsGsGp (XIV)
GsGsGsGsGsGsGsGp (XV)
wherein each G and p have the meaning indicated, and s is the thiophosphoric acid residue which forms the phosphorothioester bonding with neighboring G and each s forms the ester bondings with 3'-hydroxyl of the left side G and with 5'-hydroxyl of the right side G in the formulae respectively.

4. An anti-HIV agent which comprises as an active ingredient one or more species selected from phosphorothioester bonding type oligoribonucleotides represented by either of the below formulae (XVI)-(IXX)
pGsGsG (XVI)
pGsGsGsG (XVII)
pGsGsGsGsG (XVIII)
pGsGsGsGsGsG (IXX)
wherein G, p and s have the meaning indicated respectively.

5. An anti-HIV agent which comprises as an active ingredient one or more species selected from phosphorothioester bonding type oligoribonucleotides represented by either of the below formulae (XX)-(XXIII)
GsGsG (XX)
GsGsGsG (XXI)
GsGsGsGsG (XXII)
GsGsGsGsGsG (XXIII)
wherein G and s have the meaning indicated respectively.
